# EUROPEAN PATENT SPECIFICATION

(11) **EP 1 338 284 B1**
(45) Date of publication and mention of the grant of the patent: **27.09.2006**
(21) Application number: 01905835.3
(22) Date of filing: 22.02.2001
(51) Int. Cl.: A61K 33/14, A61K 33/10

(54) **COMPOSITION COMPRISING SODIUM, POTASSIUM, CALCIUM AND MAGNESIUM**
ZUSAMMENSETZUNG AUS NATRIUM, KALIUM, CALCIUM UND MAGNESIUM
COMPOSITION COMPRENANT DU SODIUM, DU POTASSIUM, DU CALCIUM ET DU MAGNESIUM

(30) Priority: 27.11.2000 ES 200002833
(43) Date of publication of application: 27.08.2003
(73) Proprietor: Recuperation Electrolitos, S.L., E-08036 Barcelona (ES)
(72) Inventor: BLASI ESCUDE, Alfred, E-43205 Reus (Tarragona) (ES)
(74) Representative: Carpintero Lopez, Francisco
(86) International application number: PCT/ES2001/000067
(87) International publication number: WO 2002/041905

(56) References cited:
- EP-A- 0 358 369
- EP-A- 0 587 972
- WO-A-99/37170
- ES-T- 2 144 731
- NL-C- 1 010 786
- US-A- 4 404 192
- US-A- 5 759 586
- PATENT ABSTRACTS OF JAPAN & JP 2000 060506 A (OGIWARA FUMITAKE NISHIO) 29 February 2000

## Description

### FIELD OF THE INVENTION

The invention relates to a composition which includes sodium, potassium, calcium and magnesium, and to its applications in the treatment of diseases with a muscular, neuromuscular or osseous etiology, as well as in the treatment of states lacking or deficient in said elements, and in the muscular recovery of healthy persons and animals.

### BACKGROUND OF THE INVENTION

There exist several diseases with a muscular or neuromuscular etiology which affect the muscles, generating multiple symptoms, many of which are disabling for the patient suffering them and lack specific treatment. Diseases of this type have as their main symptoms (albeit not the only ones) generalized muscular pain, muscle cramps, sleep disorders, irritable colon, headaches, visual disorders, memory loss, etc. Illustrative examples of this type of diseases include: chronic fatigue syndrome, in which the patient feels absolute exhaustion for 24 hours a day, in all cases at a muscular level; chronic pain; myofascial pain; growing pains; and above all, fibromyalgia.

The treatment used normally for these diseases consists of a symptomatic treatment by administering analgesics (magnesium metamizol, tramadol, methadone chlorhydrate, etc.); antidepressants (amitryptiline, etc.) to treat the depression associated to chronic and painful diseases, and the alteration of neurotransmitters (such as serotonin); muscle relaxants (cyclobenzaprine, diclofenac sodium, etc.) to treat muscle cramps; sedatives and sleeping pills (diazepam, potassium chlorazepate, clonazepam, etc.) to treat sleep disorders; anti-diarrheics (loperamide, etc.) to treat irritable colon, etc.

NL-C-1010786 discloses a drink comprising sodium, potassium, calcium and magnesium ions.

There is therefore a need to develop a safe and effective treatment for this type of diseases. The invention provides a composition that is useful, among other things, for the treatment of these diseases, comprising sodium, potassium, calcium and magnesium.

Sodium (Na), together with potassium, adjusts the balance of fluids in the organism, contributes to the digestive process by maintaining a suitable osmotic pressure and by acting inside cells participates in conduction of nerve impulses. A sodium deficiency is rare, as it is present in nearly all foods as a natural ingredient or as an ingredient added during elaboration, but if a sodium deficiency does occur it is manifested by dehydration, dizziness and low blood pressure. Sodium losses may be due to diarrhea, vomiting and excessive transpiration. Additionally, the use of diuretics for weight loss, sauna and intense physical activity in hot weather can lead to loss of fluids that are not compensated by a simple intake of water, and in fact this can be harmful, requiring the intake of sodium enriched liquids.

Potassium (K) boosts the activity of the kidneys aiding the elimination of toxins, is essential in storage of carbohydrates and their later conversion into energy, helps maintain a correct heartbeat rate and a normal blood pressure and is an essential mineral for transmission of nerve impulses. Potassium deficient states of the organisms lead to muscular weakness, fatigue, dizziness and confusion. Potassium and calcium are closely related, as they are both involved in controlling and managing body water levels, required for a correct muscular function.

Calcium (Ca) is an essential element for the correct development of osseous tissues. It is the mineral which forms the human skeleton and keeps it healthy and strong. Approximately 90% of the calcium in the organism of human beings is in teeth and bones, while the remaining 1% is in blood and tissues and is essential for health and life as without this small amount of calcium muscles could not contract correctly, blood would not clot and nerve endings would not be able to transport messages correctly, favoring the appearance of bone and muscle diseases. Human beings obtain this approximately 1% of the calcium required for normal functions from calcium ingested in the diet and calcium in the bones, which act as an emergency source when the body cannot find sufficient calcium in the diet. When not enough calcium is provided and the organism overuses this emergency calcium supply bone weaken and break easily (osteoporosis). A calcium rich and balanced diet and physical exercise are some solutions for preventing these problems.

Magnesium (Mg) is the fourth most important intercellular cation of the body. It plays a crucial role in muscle contraction and relaxation, improves cardiovascular health,, activates a large number of enzymes and participates in many metabolic reactions (nearly all enzymatic reactions involved in the use of ATP), it is the primary regulator of electrical activity, maintains bones, cartilage and teeth in good condition, regulates replication of nucleic acids inside the cell, plays a basic role in achieving pregnancy and preventing abortions, increases intestinal flora and allows absorption of nutrients, scars wounds and its use is recommended during pregnancy and nursing. In addition, magnesium reduces the absorption of cholesterol, eliminates excess sodium and calcium, helps potassium enter cells and feeds the nervous, muscular, neuromuscular and glandular systems, and therefore combats the effects of stress, neuromuscular hyperexcitability, signs of tetany and epileptic fits. The pituitary gland needs magnesium, and when it lacks this element it cannot control the adrenal glands, which segregate more adrenaline and make the person feel unwell, increasing heart rate, releasing glucose from the liver and causing anxiety, hyperactivity and an excessive muscular response, the person possibly becoming violent and possibly suicidal.

A correct administration of magnesium eliminates many possibilities of suffering from the following ailments: hypertension, arthritis, arthrosis, arteriosclerosis, hepatitis, cirrhosis, cancer, parathyroid diseases, insomnia, neuropsychiatric disorders, aggressiveness, anxiety, hypermotivation, nervous excitability, tachycardia, cramps, muscle contractions, growth disorders, osteoporosis, rickets, infections, rheumatic pain, neuritis, senile states, obesity, constipation, anorexia, nausea, vomiting, shaking, spasms, lethargy, personality changes, weakness, influenza, migraine, asthma and menstrual colic. Diabetics require Mg supplements. In addition, magnesium sulfate significantly reduces the risk of cerebral palsy and mental retardation, a situation which occurs mainly in underweight newborns.

Bones store approximately 55% of the body's magnesium. About 44% is inside cells, and only approximately 1% is in extracellular fluids and blood serum.

Magnesium deficiency causes muscular and nervous irritability, weakness, tiredness, fatigue, depression, low spirits, hypertension, convulsions, panic and anxiety attacks, and release of substances which favor allergies. Hen magnesium is lacking there is an increased risk of suffering a heart attack. Kidney and gall bladder stones are greatly due to a lack of magnesium, which fixes the calcium preventing the formation of calcium oxalates and phosphates, the stone components.

A high consumption of phosphorus, calcium and vitamin D supplements should be accompanies by magnesium. Most people do not ingest magnesium in sufficient amounts, as on one hand foods contain ever lower amounts of magnesium chloride, and on the other a substantial amount of the magnesium present in vegetables is lost with cooking. Stress, excessive consumption of sugar, alcohol, drugs and diuretics cause great elimination of magnesium. Furthermore, excessive dairy products, birth control pills, antibiotics, fluoride, tobacco or cortisone hinder the absorption of magnesium. Magnesium is easily excreted in faeces and urine.

### SUMMARY OF THE INVENTION

The invention deals with the problem of developing safe and effective treatment for diseases of muscular, neuromuscular or osseous etiology.

The solution provided by this invention is based on the observation by the inventor that administration to patients suffering from diseases of muscular, neuromuscular or osseous etiology of a composition comprising sodium, potassium, calcium and magnesium in adequate amounts, generally sufficient to satisfy the need of these elements in muscle cells and to maintain their adequate equilibrium, caused a fast improvement of all or most symptoms related to this type of diseases. The solution provided by the invention unlike symptomatic treatments provided by the state of the art, intends to solve the problem faced from the origin of the disease itself.

Example 1 describes the elaboration of a composition provided by this invention, the effectiveness of which in the treatment of fibromyalgia, cervicalgia, lumbalgia, arthrosis and osteoporosis has been made manifest in the tests described in Example 2.

### DETAILED DESCRIPTION OF THE INVENTION

The invention provides a composition, hereinafter referred to as the composition of the invention, which comprises a source of sodium, a source of potassium, a source of calcium and a source of magnesium.

The source of sodium comprises at least one physiologically acceptable compound, soluble or dispersible in an aqueous medium, which in solution or suspension in said aqueous medium provide sodium ions. In a particular embodiment the source of sodium is selected from among sodium chloride, a sodium citrate (mono-, di- or tri-sodium citrate) and their mixtures.

The source of potassium comprises at least one physiologically acceptable compound, soluble or dispersible in an aqueous medium, which in solution or suspension in said aqueous medium provide potassium ions. In a particular embodiment, the source of potassium comprises potassium chloride.

The source of calcium comprises at least one physiologically acceptable compound, soluble or dispersible in an aqueous medium, which in solution or suspension in said aqueous medium provide calcium ions. In a particular embodiment, the source of calcium comprises a calcium phosphate, mono-, di- o tricalcium phosphate, and their mixtures, preferably tri-calcium phosphate.

The source of magnesium comprises at least one physiologically acceptable compound, soluble or dispersible in an aqueous medium, which in solution or suspension in said aqueous medium provide magnesium ions. In a particular embodiment, the source of magnesium comprises magnesium carbonate.

The invention provides a composition characterized in that it contains:
a) a source of sodium, selected from among mono-, di-or tri-sodium citrate, sodium chloride and their mixtures;
b) a source of potassium consisting of potassium chloride;
c) a source of calcium, selected from among mono-, di-or tri-calcium phosphate and their mixtures; and
d) a source of magnesium consisting of magnesium carbonate;
in an amount such that in solution or suspension in an aqueous medium it presents, per liter of solution or suspension, the following concentration of ions:

| | |
|---|---|
| Sodium | 1.48 g |
| Potassium | 0.40 g |
| Calcium | 0.03 g |
| Magnesium | 0.03 g |

In this embodiment the composition of the invention presents the following ion distribution:
4Na⁺ + K⁺ + 3Ca²⁺ + Mg²⁺ + 2Cl⁻ + C₆H₅O₇³ + CO₃²⁻ + 2PO₄³⁻
where the citrate, carbonate and phosphate groups are the result of salts used as the sources of sodium, potassium, calcium and magnesium, although because of their properties they aid in the improvement of the symptoms related to diseases of muscular, neuromuscular or osseous etiology.

The composition of the invention can be obtained by conventional means, such as by mixing the sources of sodium, potassium, calcium and magnesium in adequate amounts in an aqueous medium which facilitates the solution or suspension of said ion sources. The aqueous medium is preferably water.

The composition of the invention can be used for both therapeutic applications and non-therapeutic ones. Among the therapeutic applications is its use to treat diseases of muscular, neuromuscular or osseous etiology, which directly or indirectly affect muscles and bones, such as fibromyalgia, chronic pain, myofascial pain, rheumatism, rheumatic arthritis, hemorrhoids, lumbago, gout, headaches of a neuromuscular origin, static vertebral alterations, growing pains, diarrheas and constipation, arthrosis, cervicalgias, etc., as well as in the treatment of deficiency states of sodium, potassium, calcium and/or magnesium.

It is believed that the technical effect provided by the composition of the invention is attained by providing the organism's cells with a supply of ions which they lack or in which they are deficient. The concentration gradient produced must be maintained, as this difference in concentration causes a potential difference which allows conduction of impulses and results in muscular contraction. By means of the composition of the invention it is possible to make muscle cells maintain a correct inner concentration of said ions.

The invention therefore provides a pharmaceutical composition which comprises a therapeutically effective amount of a composition of the invention together with at least one pharmacologically acceptable excipient.

The pharmaceutical compositions contained in the composition of the invention can be presented in any form of administration, such as solid (pills, granules, powders, capsules, lotions, suppositories, etc.) or liquid (drops, injections, syrups, etc.) all of which can be manufactured by conventional methods and administrated by any appropriate means such as orally, parenterally or topically, for which they will include pharmacologically acceptable excipients required for formulating the desired form of administration (agglutinants, disintegrators, thickeners, dispersants, promoters of reabsorption, correctors, buffers, tensoactives, solubilizers, preservatives, emulsifiers, isotonic agents, stabilizers, pH adjusting agents, etc. as required). A review of the various pharmaceutical means of administering medicaments and the required excipients to obtain them can be found for example in "*Tratado de Farmacia Galénica*" "Treatise on Galenical Pharmacy", C. Fauli i Trillo, 1993, Luzán 5, S.A. Editions, Madrid.

In a specific embodiment the pharmaceutical composition provided by this invention is presented in a solid form of administration which can be administered orally as such or dissolved or suspended in an aqueous medium, preferably water. In another specific embodiment, the pharmaceutical composition provided by this invention is presented in the form of an aqueous solution or suspension suitable for oral administration. The composition of the invention, presented in any form of presentation, maintains the muscular stabilization, anticramping, analgesic and palliative properties of the symptoms characteristic of diseases of a muscular, neuromuscular or osseous etiology, as well as its properties of supplying the elements sodium, potassium, calcium and/or magnesium, in order to treat states lacking or deficient in said elements, thereby improving the symptoms characteristic of these diseases or pathologic disorders.

The dose of the composition of the invention to be administered depends on the specific type of the composition, the age and weight of the patient, the form of dosification employed, the type and stage of the disease and other conditions. However, given the innocuousness of the product and the great tolerance described of all its components an overdose is highly unlikely.

The invention also relates to the use of a composition of the invention in a medicament for treating diseases of a muscular, neuromuscular or osseous etiology and which directly and indirectly affect muscles and bones, such as fibromyalgia, chronic pain, myofascial pain, rheumatism, rheumatoid arthritis, hemorrhoids, lumbago, gout, headaches of a neuromuscular origin, static vertebral alterations, growing pains, diarrheas and constipation, arthrosis, cervicalgias, etc., or for treatment of states lacking or deficient in sodium, potassium, calcium and/or magnesium.

The invention further provides the use of the composition of the invention in the elaboration of a medicament for the treatment of diseases of a muscular, neuromuscular or osseous etiology, or states lacking or deficient in sodium, potassium, calcium and/or magnesium.

Among the non-therapeutic applications of the composition of the invention is its use for muscular recovery of healthy persons and animals, such as to compensate muscular efforts made by athletes or workers who perform intense muscular activity, thereby aiding muscular recovery but without seeking a therapeutic objective.

The composition of the invention, for these non-therapeutic applications, can be presented in any suitable form of administration, such as solid, for administration as such or after solution or suspension in an aqueous medium, or liquid such as in the form of an aqueous solution or suspension.

Unless it opposes the object of the invention, the composition of the invention may contain suitable amounts of other therapeutically active ingredients.

The following examples serve to illustrate the invention and should not be understood in a limiting sense.

### EXAMPLE 1

### Elaboration of a composition of the invention

A composition of the invention was prepared containing sodium chloride, tri-sodium citrate, potassium chloride, tri-calcium phosphate and magnesium carbonate, in amounts such that in an aqueous solution it provided the following amounts of ions per liter of solution:

| Ion | g/l |
|---|---|
| Sodium | 1.48 |
| Potassium | 0.40 |
| Calcium | 0.03 |
| Magnesium | 0.03 |
| Chloride | 0.70 |

For this purpose the required amounts were weighed of each salt and mixed in a suitable vessel and a suitable amount of water was added. An aqueous solution was obtained of the composition of the invention which was used in the following trials.

### EXAMPLE 2

### Efficacy of the composition of the invention

The efficacy of the composition of the invention described in Example 1 was tested on patients affected with (i) fibromyalgia, (ii) cervicalgia and lumbalgia, and (iii) arthrosis and osteoporosis.

### 1. Efficacy in patients affected with fibromyalgia

The composition described in example 1 was administered orally to volunteer patients affected with severe fibromyalgia for one month in a dose of 1.250 ml daily.

All patients found clear a improvement in the first 36 hours after the first ingestion. After 48 hours certain symptoms began to disappear gradually, until some of them practically disappeared. Persisting symptoms were diminished to fully bearable levels, not ruling out their full disappearance in a nearby future.

These results provide hope for patients with a chronic disease without specific treatment, without a known etiology and with a high level of suffering by those who suffer it.

### 2. Efficacy in patients with cervicalgias and lumbalgia

A group of volunteer patients affected with chronic cervicalgias and lumbagos (selected for belonging to a specific risk group, hairdressers) were orally administered for one month 500 ml daily of the composition described in Example 1.

Absolutely all patients showed a full disappearance of the muscle knots which caused cervicalgia and an obvious decrease in lumbar discomfort. Persisting and only in part were discomfort in patients with physical disorders such as pinched nerves or herniated discs.

### 3. Efficacy in patients with arthrosis and osteoporosis

A group of volunteer men and women over 50 years of age, affected with varying degrees of arthrosis and osteoporosis, cervicalgias and chronic lumbago were uninterruptedly orally administered for 2 months with 1,000 ml daily of the composition described in Example 1.

All volunteers showed improvement in the symptoms caused by their arthrosis and/or osteoporosis, with the improvement most obvious as regards the perception of pain and discomfort for perambulation than in the analytical test of said recovery.

## Claims

1. A composition comprising:
a) a source of sodium which comprises a physiologically acceptable compound, soluble or dispersible in an aqueous medium which, in solution or suspension in said aqueous medium, provides sodium ions;
b) a source of potassium which comprises a physiologically acceptable compound, soluble or dispersible in an aqueous medium which, in solution or suspension in said aqueous medium, provides potassium ions;
c) a source of calcium which comprises a physiologically acceptable compound, soluble or dispersible in an aqueous medium which, in solution or suspension in said aqueous medium, provides calcium ions; and
d) a source of magnesium which comprises a physiologically acceptable compound, soluble or dispersible in an aqueous medium which, in solution or suspension in said aqueous medium, provides magnesium ions,
**characterized in that** when said composition is solved or dispersed in an aqueous medium it originates an aqueous solution or suspension which presents per litre of solution or suspension: 1.48 g of sodium ions, 0.40 g of potassium ions, 0.03 g of calcium ions and 0.03 g of magnesium ions.

2. Composition according to claim 1 **characterized in that**:
a) the source of sodium is selected from mono-, di- or tri-sodium citrate, sodium chloride, and their mixtures;
b) the source of potassium is selected from potassium chloride or potassium citrate;
c) the source of calcium is selected from mono-, di-or tricalcium phosphate and their mixtures; and
d) the source of magnesium is magnesium carbonate;

3. Composition according to claims 1-2 for use in medicine.

4. Composition according to claims 1-2 for the treatment of fibromyalgia, chronic pain, myofascial pain, rheumatism, rheumatoid arthritis, hemorrhoids, lumbago, gout, headaches of a neuromuscular origin, static vertebral alterations, growing pains, diarrheas, constipation, arthrosis or cervicalgias.

5. Use of a composition according to claims 1-2 in the elaboration of a medicament for the treatment of fibromyalgia, chronic pain, myofascial pain, rheumatism, rheumatoid arthritis, hemorrhoids, lumbago, gout, headaches of a neuromuscular origin, static vertebral alterations, growing pains, diarrheas, constipation, arthrosis or cervicalgias.

6. Use of a composition according to claims 1-2 for the muscular recovery of healthy persons and animals.

## Patentansprüche

1. Zusammensetzung, umfassend:
a) eine Natriumquelle, die eine in einem wässrigen Medium lösliche oder dispergierbare physiologisch unbedenkliche Verbindung umfasst, die in dem wässrigen Medium gelöst oder suspendiert Natriumionen liefert;
b) eine Kaliumquelle, die eine in einem wässrigen Medium lösliche oder dispergierbare physiologisch unbedenkliche Verbindung umfasst, die in dem wässrigen Medium gelöst oder suspendiert Kaliumionen liefert;
c) eine Calciumquelle, die eine in einem wässrigen Medium lösliche oder dispergierbare physiologisch unbedenkliche Verbindung umfasst, die in dem wässrigen Medium gelöst oder suspendiert Calciumionen liefert;
d) eine Magnesiumquelle, die eine in einem wässrigen Medium lösliche oder dispergierbare physiologisch unbedenkliche Verbindung umfasst, die in dem wässrigen Medium gelöst oder suspendiert Magnesiumionen liefert,
**dadurch gekennzeichnet, dass** die Zusammensetzung, wenn sie in einem wässrigen Medium gelöst oder dispergiert ist, eine wässrige Lösung oder Suspension ergibt, die pro Liter Lösung oder Suspension aufweist: 1,48 g Natriumionen, 0,40 g Kaliumionen, 0,03 g Calciumionen und 0,03 g Magnesiumionen.

2. Zusammensetzung nach Anspruch 1, **dadurch gekennzeichnet, dass**
a) die Natriumquelle gewählt ist aus Mono-, Di- oder Trinatriumcitrat, Natriumchlorid und deren Mischungen;
b) die Kaliumquelle gewählt ist aus Kaliumchlorid oder Kaliumcitrat;
c) die Calciumquelle gewählt ist aus Mono-, Di- oder Tricalciumphosphat und deren Mischungen; und
d) die Magnesiumquelle Magnesiumcarbonat ist.

3. Zusammensetzung nach den Ansprüchen 1 - 2 zur Verwendung in der Medizin.

4. Zusammensetzung nach den Ansprüchen 1-2 zur Behandlung von Fibromyalgie, chronischen Schmerzen, myofaszialen Schmerzen, Rheumatismus, rheumatoider Arthritis, Hämohorriden, Lumbago, Gicht, Kopfschmerzen neuromuskulären Ursprungs, statischer Wirbelveränderungen, Wachstumsschmerzen, Durchfällen, Verstopfungen, Arthrosen oder Zervikalgien.

5. Verwendung einer Zusammensetzung nach den Ansprüchen 1 - 2 bei der Entwicklung eines Medikaments zur Behandlung von Fibromyalgie, chronischen Schmerzen, myofaszialen Schmerzen, Rheumatismus, rheumatoider Arthritis, Hämohorriden, Lumbago, Gicht, Kopfschmerzen neuromuskulären Ursprungs, statischer Wirbelveränderungen, Wachstumsschmerzen, Durchfällen, Verstopfungen, Arthrosen oder Zervikalgien.

6. Verwendung einer Zusammensetzung nach den Ansprüchen 1 - 2 bei der Muskelerholung gesunder Personen und Tiere.

## Revendications

1. Composition comprenant :
a) une source de sodium qui comprend un composé physiologiquement acceptable, soluble ou dispersible dans un milieu aqueux qui, en solution ou en suspension dans ledit milieu aqueux, fournit des ions sodium ;
b) une source de potassium qui comprend un composé physiologiquement acceptable, soluble ou dispersible dans un milieu aqueux qui, en solution ou en suspension dans ledit milieu aqueux, fournit des ions potassium ;
c) une source de calcium qui comprend un composé physiologiquement acceptable, soluble ou dispersible dans un milieu aqueux qui, en solution ou en suspension dans ledit milieu aqueux, fournit des ions calcium ; et
d) une source de magnésium qui comprend un composé physiologiquement acceptable, soluble ou dispersible dans un milieu aqueux qui, en solution ou en suspension dans ledit milieu aqueux, fournit des ions magnésium ;
**caractérisée en ce que** lorsque ladite composition est dissoute ou dispersée dans un milieu aqueux, elle devient une solution ou suspension aqueuse qui présente par litre de solution ou de suspension : 1,48 g d'ions sodium, 0,40 g d'ions potassium, 0,03 g d'ions calcium et 0,03 g d'ions magnésium.

2. Composition selon la revendication 1, **caractérisée en ce que** :
a) la source de sodium est sélectionnée parmi le mono-, di- ou tri- citrate de sodium, le chlorure de sodium, et leurs mélanges ;
b) la source de potassium est sélectionnée parmi le chlorure de potassium ou le citrate de potassium ;
c) la source de calcium est sélectionnée parmi le mono-, di- ou tri- phosphate de calcium et leurs mélanges ;
d) la source de magnésium est du carbonate de magnésium.

3. Composition selon la revendication 1 ou 2 destinée à être utilisée en médecine.

4. Composition selon la revendication 1 ou 2, destinée au traitement de la fibromyalgie, la douleur chronique, la douleur myofasciale, le rhumatisme, la polyarthrite rhumatoïde, les hémorroïdes, le lumbago, la goutte, les maux de tête d'origine neuromusculaire, les altérations vertébrales statiques, les douleurs de croissance, les diarrhées, les constipations, l'arthrose ou les cervicalgies.

5. Utilisation d'une composition selon la revendication 1 ou 2 pour la fabrication d'un médicament destiné au traitement de la fibromyalgie, la douleur chronique, la douleur myofasciale, le rhumatisme, la polyarthrite rhumatoïde, les hémorroïdes, le lumbago, la goutte, les maux de tête d'origine neuromusculaire, les altérations vertébrales statiques, les douleurs de croissance, les diarrhées, les constipations, l'arthrose ou les cervicalgies.

6. Utilisation d'une composition selon la revendication 1 ou 2 pour la récupération musculaire de personnes et d'animaux sains.
